# FASCICULE DE BREVET EUROPEEN

(11) **EP 1 165 600 B1**
(45) Date de publication et mention de la délivrance du brevet: **04.08.2004**
(21) Numéro de dépôt: 00915218.2
(22) Date de dépôt: 23.03.2000
(51) Int. Cl.: C07K 2/00, A61K 47/48

(54) **PROTEINES MODIFIEES PAR L'ACIDE XANTHURENIQUE**
DURCH XANTHURENSÄURE MODIFIZIERTE PROTEINE
PROTEINS MODIFIED BY XANTHURENIC ACID

(30) Priorité: 26.03.1999 FR 9903791
(43) Date de publication de la demande: 02.01.2002
(73) Titulaire: Malina, Halina, 94940 Les Ulis (FR)
(72) Inventeur: Malina, Halina, 94940 Les Ulis (FR)
(86) Numéro de dépôt international: PCT/FR2000/000757
(87) Numéro de publication internationale: WO 2000/058344

(56) Documents cités:
- MALINA, HALINA ZOFIA ET AL: "Xanthurenic acid derivative formation in the lens is responsible for senile cataract in humans" GRAEFE'S ARCH. CLIN. EXP. OPHTHALMOL., vol. 234, no. 12, décembre 1996 (1996-12), pages 723-730, XP000867409 cité dans la demande
- KOTAKE Y ET AL: "The physiological significance of the xanthurenic acid- insulin comples." JOURNAL OF BIOCHEMISTRY, vol. 77, no. 3, mars 1975 (1975-03), pages 685-687, XP000867442
- MURAKAMI E: "Purification of xanthurenic acid-insuline complex." ACTA VITAMINOLOGICA ET ENZYMOLOGICA, vol. 29, no. 1-6, 1975, pages 240-242, XP000867444
- KOBAYASHI K ET AL: "Influence of blood proteins on biomedical analysis. I. Interaction of xanthurenic acid with bovine serum albumin." CHEMICAL AND PHARMACEUTICAL BULLETIN, vol. 28, no. 10, octobre 1980 (1980-10), pages 2960-2966, XP002128451
- MALINA H Z: "Xanthurenic acid provokes formation of unfolded proteins in endothelial reticulum of the lens epithelial cells" BIOCHEMICAL AND BIOPHYSICAL RESEARCH COMMUNICATIONS, vol. 265, no. 2, 19 novembre 1999 (1999-11-19), pages 600-605, XP000867480
- SHIRAO Y ET AL.: "Glucoside of xanthurenic acid accumulates in brunescent but not in non-brunescent lens nuclei in human" IOVS, vol. 40, no. 4, 15 mars 1999 (1999-03-15), page S 522 XP000907486 1999 Annual Meeting of the Association for Research in Vision and Ophtalmology, 9-14/5/1999, Fort Lauderdale, FL

## Description

La présente invention à pour but l'induction d'une réponse immunitaire contre les pathologies induites par des modifications de physiologie cellulaire par l'acide xanthurénique. La présente invention concerne aussi une façon d'induction régulée de pathologie cellulaire en présence de l'acide xanthurénique. Elle est relative à une formation de protéines modifiées de façon covalente par l'acide xanthurénique *in vitro* ou dans un système cellulaire, et non pas de façon non-covalente comme decrit précedement (Kotake Y. et al. J. Biochem. 1975, 77, 685-687; Kobayashi K. et al. Chem. Pharm. Bull. 1980. 28, 2960-2966). La base de l'invention est une observation du fait que Façade xanthurénique mène à la modifi-cation covalente de protéines dans des cellules et provoque une modification de la physiologie cellulaire. Précédemment, il a été reporté que l'acide xanthurénique s'accumule dans le cristallin de l'oeil bovin (Malina et al. Graefe's Arch. Clin. Exp. Ophthalmol. 1995, 233, 38-44), et humain (Malina et al. Graefe's Arch. Clin. Exp. Ophthalmol. 1996, 234, 723-730) avec l'âge, dans sa présence les α, β, γ-cristallines forment des agrégats (idem) et deviennent fluorescentes (Maline et al. Eur. J. Ophthalmol, 1996, 6, 250-256). Les conjugués covalents sont formés par la préparation des produits oxydés de l'acide xanthurénique, nommés DOXA et sa réaction avec des cristallines de l'oeil (Malina et al. Graefe's Arch. Clin. Exp. Ophthalmol. 1996, 234, 723-730). Récemment, les expériences ont montré que l'acide xanthurénique s'accumulant dans une cellule conduit à une modification de la physiologie cellulaire.

Cette modification est due à une accumulation de protéines mal repliées. L'acide xanthurénique peut former des liaisons covalentes avec des protéines. En présence de l'acide xanthurénique qui a une couleur jaune, des protéines deviennent jaunes à cause de celui-ci. Cette couleur persiste après l'électrophorèse des protéines sur le gel dénaturant. Ces résultats montrent que l'acide xanthurénique est lié avec les protéines de façon covalente. Pour changer la conformation d'une protéine, il est suffisant de modifier un acide aminé; de nombreux exemples sont présents dans la littérature scientifique. En présence de l'acide xanthurénique comme l'indiquent les exemples donnés dans cette description, un à plusieurs acides aminés peuvent être modifiés. Pour cette raison, la présence de l'acide xanthurénique dans une cellule provoque une surexpression des protéines chaperonnes nommées "glucose regulated proteins 94" GRP94. La surexpression de ces protéines est connue comme étant provoquée par l'accumulation des protéines mal repliées (Kozutsumi Nature 1988,332,462-464).

L'acide xanthurénique modifie des protéines de façon aléatoire et cette modification concerne aussi les protéines chaperonnes comme par exemple la GRP 94 et la calréticuline. Etant donné que ces protéines sont responsables de la conformation correcte des protéines, leur modification accélère l'accumulation des protéines mal repliées et aussi parmi elles des immunoglobulines mal repliées. Ces modifications complexes des protéines par l'acide xanthurénique, permettent de laisser fonctionner des cellules avec une physiologie modifiée. L'accumulation des protéines modifiées par l'acide xanthurénique dans différents types de cellules (par exemple les cellules des astrocytes, les cellules épithéliales du cristallin) provoque par exemple une surexpression des proteases, une dégradation de la calréticuline, une modification du facteur nucléaire-kappaB, et une induction du β-amyloïde (A4).

Ces résultats montrent que la formation de protéines modifiées par l'acide xanthurénique mène à une pathologie cellulaire en induisant le changement de nombreuses protéines. Les changements observés sont en fonction du degré de la modification de protéines par l'acide xanthurénique.

Ces résultats montrent qu'il est possible d'induire une pathologie cellulaire de façon artificielle en augmentant dans les cellules le niveau de protéines modifiées par l'acide xanthurénique. Ce nouveau mécanisme est provoqué par la modification des protéines par l'acide xanthurénique dans une cellule. Dans une culture cellulaire des astrocytes une augmentation du niveau de protéines modifié par l'acide xanthurénique provoque l'induction de β-amyloïde (A4), qui est reconnu par les anticorps monoclonaux de Dako, Danemark, utilisés pour le diagnostic de la maladie d' Alzheimer. La raison de cette induction de β-amyloïde est une modification de la conformation de la protéine précurseur de l'amyloïde (PPA), due à la modification par l'acide xanthurénique. Cette modification donne le signal à une induction de proteases, qui dégradent le PPA modifié et induisent la formation de β-amyloïde (A4). L'acide xanthurénique est un acide aminé de la voie de dégradation du tryptophane et son accumulation dans différents types de cellules peut conduire à diverses pathologies. On peut prévoir que l'animal dans lequel on augmente le niveau de protéines modifiées par l'acide xanthurénique peut servir comme modèle pour étudier l'effet de médicaments.

Une introduction directe d'acide xanthurénique par voie orale ou d'autres voies, peut servir comme un modèle de développement de la maladie d'Alzheimer, des maladies à priones, de la cataracte sénile, de l'athérosclérose, des rhumatismes, de la dégénérescence de la rétine avec l'âge.

L'observation du fait que l'acide xanthurénique provoque une dérégulation de la physiologie cellulaire permet une induction régulée de la pathologie cellulaire.

Des protéines modifiées par l'acide xanthurénique et injectées à un animal vont induire une réponse immunitaire contre les protéines mal repliées. A cause de la modification du système immunitaire par l'acide xanthurénique et à la suite d'une dégradation des protéines chaperonnes comme la GRP94, les cellules pathologiques ne sont pas éliminées. L'induction de la réponse immunitaire contre les protéines mal repliées peut prévenir l'effet pathologique qui a lieu à la formation de ces protéines au cours de vieillissement.

Les vaccins basés sur des protéines modifiées par l'acide xanthurénique vont avoir un rôle préventif contre les maladies induites par des protéines ainsi modifiées. Les protéines modifiées par l'acide xanthurénique peuvent être administrées aux mammifères en utilisant tous les solvants non toxique dans lesquels elles sont solubles. Les degrés de modification de la protéine par l'acide xanthurénique, et la quantité de protéine à administrer va dépendre de la protéine à modifier et du but recherché par la vaccination. Les fragments de protéines, des peptides ou des séquences synthétiques peuvent être utilisés pour former des produits conjugués avec l'acide xanthurénique. Ces composés sont introduits dans un mammifére pour induire une réponse immunitaire.

### Exemple 1. Formation de protéines modifiées par l'acide xanthurénique dans la culture de cellules epithéliales.

La culture primaire des cellules epithéliales de bovins dans un milieu du type milieu essentiel minimal (MEM) a été traitée par l'acide xanthurénique. L'acide xanthurénique a été ajouté dans ce milieu à concentration 0,1, 2, 4 mM. Après 24 heures de culture les cellules ont été lavées en utilisant un tampon PBS (5 mM sodium phosphate, 150 mM NaCl, pH 7.1) et lysées dans un tampon contenant 50 mM Tris-HCl (pH8), 150 mM NaCl 100 µg/ml PMSF, 1% Triton X-100. Des extraits ont été appliqués sur une colonne de Sephadex G-50 et élués en utilisant 0,005 M NaHCO3. L'acide xanthurénique a été quantifié dans les extraits de protéines par la spectrométrie UV. La concentration de protéines a été calculée en utilisant une gamme étalonnée des mesures de l'absorption des quantités connues d'albumine du bovin ayant le poids moléculaire de 67,5 kD après une incubation avec l'acide xanthurénique λ=342 nm (E_{λmax} 6 500 selon Merck Index, Merck and Co., édition White House Station, New York, 1996). La concentration de l'acide xanthurénique a correspondu respectivement à 0, 1, 3, 9 moles par mole de protéines. Les analyses des protéines après un transfert de gel SDS-PAGE sur une membrane de nylon (Western blot) en présence des différents anticorps ont montré qu'en présence de protéines modifiées par l'acide xanthurénique le niveau de facteur nucléaire κB, β-amyloïde (A4), et calpain Lp82 ont été changés.

### Exemple 2

### Formation de protéines modifiées par l'acide xanthurénique dans la culture cellulaire des astrocytes.

La culture d'astrocytes de rat dans le milieu MEM a été traitée par l'acide xanthurénique à concentration 0, 2, 4, 8 mM. La concentration de l'acide xanthurénique (XA) dans des extraits a été calculée comme dans l'exemple 1, et a correspondu respectivement au 0 ; 1 mole XA par 8 moles de protéines ; 3 moles de XA par 2 moles de protéines ; 1 mole XA par 5 moles de protéines.

En présence de protéines modifiées par l'acide xanthurénique, le facteur nucléaire -κB a eu les poids moléculaires de 50 kD, 52kD, et 55 kD au lieu de la taille normale 50 kD. La formation β-amyloïde (A4), qui n'était pas détectable sans la présence de l'acide xanthurénique, a été fortement induite. Ces résultats ont montré qu'une augmentation de l'acide xanthurénique dans la cellule va provoquer une dérégulation de la physiologie cellulaire. Ces résultats montrent qu'il est possible d'induire artificiellement une pathologie cellulaire en augmentant dans une cellule le niveau des protéines modifiées par l'acide xanthurénique. Le nouveau mécanisme décrit est provoqué par la modification covalente des protéines par l'acide xanthurénique.

### Exemple 3. Formation de protéines modifiées par l'acide xanthurénique dans un extrait cellulaire de la rétine.

L'acide xanthurénique à 0, 2, 4, 8 mM a été incubé avec des extraits de protéines de la rétine pendant une semaine et les extraits ont été traités comme décrit dans l'exemple 1, et les concentrations ont correspondu respectivement à 0; 2 mole XA par 1 mole de protéines; 3 moles de XA par 1 mole de protéines; 5 moles XA par moles de protéines.

### Exemple 4.

### Formation de protéines modifiées par l'acide xanthurénique dans la culture de tissus.

Les cristallins de porc ont été incubés dans les solutions d'acide xanthurénique 0 et 2 mM pendant une semaine. L'acide xanthurénique a été diffusé dans le cristallin. Le cortex du cristallin a été homogénéisé dans un tampon phosphate de 7.4. La partie non soluble de protéines à été séparée par centrifugation à 10 000g. La concentration des protéines a été mesurée à 280 nm, les parties insolubles des protéines ont été dissoutes dans 4 mM urée ou dans 8 mM d'urée. L'acide xanthurénique était présent dans tous les extraits et sa quantité a augmenté avec l'insolubilité des protéines : les concentrations en acide xanthurénique dans les protéines correspondaient à 1 mole XA pour 1 mole de protéines dans la partie soluble du tampon phosphate, 2 moles de XA dans les protéines solubles dans 4mM d'urée, et 3 moles de XA dans les protéines solubles dans 8 mM d'urée.

### Exemple 5. Préparation des conjugués de l'acide xanthurénique avec des protéines de bactéries.

Le mycelium de *Streptomyces incarnatus,* une bactérie mycelial Gram-positive, a été cultivé en l'absence ou en présence de 2 mM d'acide xanthurénique. 100 ml de chaque culture a été suspendue dans le tampon de phosphate à concentration 0.05 M, de pH 7, contenant 0.1% de β-mercaptoéthanol. La suspension a été congelée dans un bain-marie contenant de la glace carbonique-methanol. Les cellules congelées ont été casées dans la presse de Hinton avec une pression de 360 atmosphères. Les protéines de cytosol ont été séparées de la fraction des membranes par centrifugation à 100 000g pendant une heure.

La solution a été traitée par l'addition de 2,5 % de streptomycine pour précipiter les acides nucléiques, qui ont été éliminé par centrifugation à 5000g pendant 10 min. Les concentrations d'acide xanthurénique dans les protéines ont été mesurées comme décrit dans l'exemple 1.

Les concentrations en acide xanthurénique dans les protéines correspondaient à 0 et 0.5 mole d'acide xanthurénique pour une mole de protéines.

### Exemple 6.

### Induction d'une réponse immunitaire contre la protéine modifiée par l'acide xanthurénique.

La calréticuline est modifiée par l'acide xanthurénique dans une cellule et partiellement dégradée. 3 mg de calréticuline dans le tampon phosphate stérile de pH 7,4 ont été incubés avec 4 mM d'acide xanthurénique pendant 72 heures, à température ambiante.

La calréticuline modifiée a été administrée aux souris. Six souris (pesant 100 g environs) ont été immunisées par injection sous-cutané en utilisant les même quantités 500µg de calreticuline. Un autre groupe de souris est resté sans traitement L'immunisation a été répétée trois fois dans l'intervalle de deux semaines. La calréticuline a été analysée dans le plasma des animaux après trois mois. Des protéines de plasma de souris ont été analysées par électrophorése sur un gel dénaturant (Laemmi, Nature 1970, 227, 680-685).

Des protéines ont été transférées sur une membrane. La détection de la calréticuline a été effectuée en utilisant un anticorps contre la calréticuline. Dans le plasma de souris non traités, la calréticuline dégradée présentait un poids moléculaire de 55 kD au lieu de 63kD. Chez les souris traitées le plasma a contenu 60 pour-cent de moins de la calréticuline dégradées.

Cette voie peut être utilisée pour retarder le vieillissement pathologique des cellules dû à une modification de la conformation des protéines, notament des protéines chaperonnes.

Les injections des protéines modifiées par l'acide xanthurénique peuvent avoir un effet préventif contre les pathologies liées au vieillissement. Une immunothérapie utilisant les anticorps monoclonaux serait possible pour retarder l'effet de protéines mal repliées. Par exemple un anticorps contre la protéine précurseur d'amyloïde modifiée par l'acide xanthurénique est supposé retarder le développement de la maladie d'Alzheimer.

## Revendications

1. Composé destiné à provoquer des réactions immunitaires dans un organisme vivant **caractérisé en ce qu'**il est le produit de la réaction de l'acide xanthurénique avec une protéine, dans laquelle l'acide xanthurénique et ladite protéine se lient de façon covalente.

2. Composé selon la revendication 1 **caractérisé en ce que** l'acide xanthurénique est lié de façon covalente à une protéine, un peptide, ou une séquence de protéine.

3. Composé selon la revendication 1 **caractérisé en ce que** cette protéine est une protéine humaine ou une protéine d'un autre mammifère.

4. Composé selon ia revendication 1 **caractérisé en ce que** cette protéine est une protéine bactérienne.

5. Composé selon la revendication 1 **caractérisé en ce que** la réaction de l'acide xanthurénique avec une protéine, dans laquelle l'acide xanthurénique et ladite protéine se lient de façon covalente, est faite par incubaction de culture de cellules en presence d'acide xanthurénique.

6. Composé selon la revendication 1 **caractérisé en ce que** la réaction de l'acide xanthurénique. avec une protéine, dans laquelle l'acide xanthurénique et ladite protéine se lient de façon covalente, est faite par incubation de tissus en presence d'acide xanthurénique.

## Patentansprüche

1. Verbindung dazu bestimmt, Immunreaktionen in einem lebenden Organismus zu verursachen, und welche dadurch charakterisiert ist, dass sie ein Produkt aus der Reaktion von Xanthurensäure mit einem Protein ist, in welchem Xanthurensäure und das besagte Protein eine kovalente Bindung eingehen.

2. Verbindung von Xanthurensäure nach Forderung 1, dadurch charakterisiert ist, dass Xanthurensäure kovalent mit einem Protein, ein Peptid oder einer Proteinsequenz verbunden ist.

3. Verbindung von Xanthurensäure nach Forderung 1, dadurch charakterisiert dass dieses Protein ein menschliches Protein oder einem Protein eines anderen Säugetieres ist.

4. Verbindung von Xanthurensäure nach Forderung 1, dadurch charakterisiert ist, dass dieses Protein ein Bakterienprotein ist.

5. Verbindung von Xanthurensäure nach Forderung 1, dadurch charakterisiert ist dass die Reaktion Xanthurensäure mit einem Protein, in dem Xanthurensäure und besagtes Protein sich kovalent binden, dies ensteht durch Inkubation von Zellen der Kultur in Anwesenheit von Xanthurensäure.

6. Verbindung von Xanthurensäure nach Forderung 1, dadurch charakterisiert ist, dass die Reaktion Xanthurensäure mit einem Protein, in dem Xanthurensäure und besagtes Protein sich kovalent binden, dies ensteht durch Inkubation von Gewebe mit Xanthurensäure.

## Claims

1. Compound destined to cause immune response in a living organism being the product of the reaction between xanthurenic acid and a protein, in which the protein and xanthurenic acid are covalently linked.

2. Compound according to claim 1 **characterized that** xanthurenic acid is covalently linked to a protein, a peptide, or a protein sequence.

3. Compound according to claim 1 **characterized that** this protein is human protein or a protein of another mammal.

4. Compound according to claim 1 **characterized that** this protein is one bacterial protein.

5. Compound according to claim 1 **characterized that** the reaction between xanthurenic acid and a protein, in which xanthurenic acid and this protein are covalently linked, is made by an incubation of cells culture in a presence of xanthurenic acid.

6. Compound according to claim 1 **characterized that** the reaction between xanthurenic acid and a protein, in which xanthurenic acid and this protein are covalently linked, is made by incubation the tissue in a presence of xanthurenic acid.
